# EUROPEAN PATENT APPLICATION

(11) **EP 3 859 077 A1**
(43) Date of publication of application: **04.08.2021**
(21) Application number: 19877263.4
(22) Date of filing: 21.10.2019
(51) Int. Cl.: D06M 15/00, D06M 15/15, D06M 15/03, A61K 8/02, A61K 8/55, A61K 8/39, A61K 8/37, A61K 8/63, A61K 8/98, A61K 8/99, A61K 8/34, A61K 8/81, A61K 8/73, A61Q 19/00, A61Q 17/00, A61K 9/70

(54) **MODIFIED FABRIC HAVING SURFACE THEREOF COATED WITH MEDIUM SYSTEM, AND PRODUCTION METHOD THEREFOR**

(30) Priority: 22.10.2018 CN 201811232397
(71) Applicant: Shandong Bright Tour Agricultural Technology Co., Ltd., Zhaili Town, Nonggao District Laiwu Shandong 271121 (CN)
(72) Inventor: LI, Hewei, Shandong 271121 (CN)
(74) Representative: Sun, Yiming
(86) International application number: PCT/CN2019/112200
(87) International publication number: WO 2020/083184

(57) **Abstract**

Provided are a modified fabric having a surface thereof coated with a medium composition, and a production method thereof. The modified fabric includes a base fabric and a medium composition coated on the base fabric, the medium composition includes a medium, or includes a medium and a medium solvent capable of dissolving or dispersing the medium. Moreover, a multi-layer modified fabric having a surface thereof coated with a medium composition and a production method thereof are provided. The modified fabric is applicable to the fields such as cosmetics, daily necessities, medicines, and medical apparatus. The modified fabric also has no additives, carries a long shelf life, has stable and undamaged functional ingredients, a low production cost, and wide market adaptability.

## Description

### TECHNICAL FIELD

The present invention relates to a facial mask for skin care, and particularly to a modified fabric product having a surface coated with a medium composition and a production method thereof. After being made into a finished product and before being used by consumers, the modified fabric product is in a dry state containing little to no water.

### BACKGROUD

Commercially available products such as facial masks, external medicinal dressings, dressings belonging to medical apparatus, and wet tissues all use a certain material of base fabric as a substrate or a carrier. That material is then combined and fully mixed with a certain amount of water, medicinal, functional, or nutritional ingredients, and packaged together for the consumer's use when needed.

In such commercially available products, numerous additives, such as antiseptics, bacteriostatic agents, and the like, are generally added to the products to ensure the quality and effectiveness of their functional and pharmaceutical ingredients. However, these additives cannot fundamentally solve the problem. On the contrary, the antiseptics and bacteriostatic agents in facial masks and other products will bring a series of negative side effects, such as allergies and contact dermatitis. These negative effects will not only void protecting the skin but will also prevent the desired effects of the product. The quality of a product during its shelf life, like facial masks for example, will be difficult to guarantee if the antiseptic additives are not included.

In conclusion, the inventor focused on innovation and has conducted in depth research and experimental work. By adopting a method of coating medium composition on the surface, the inventor has developed a series of medium composition formulas that can avoid the addition of antiseptics and additives, so as to replace the above products with a novel modified fabric product. Therefore, the present invention solves the problems caused by the side effects of the additives and cost of the prior fabric products while also having a good product market and application prospect. In addition, the present invention further optimizes the series of previously-developed modified fabric products without using antiseptics and additives, especially improving upon the defects such as high production cost, long processing time and unstable efficacy of the previous modified fabric products.

### SUMMARY

In order to solve the above-mentioned problems (that existing facial masks need to be soaked in a solution containing antiseptics and additives) the present invention further optimizes the production process technology after the development of a series of modified fabrics, all the while comprehensively improving the product formula and improving the preparation process as well.

The present invention first provides a modified fabric product having a surface coated with a medium composition. The modified fabric product includes a base fabric and a medium composition coated on the base fabric. The medium composition includes a medium, and the medium simultaneously has the following properties:
A. under a normal temperature of 0°C-60°C, in a solidification state;
B. under a high temperature of 30°C-500°C, in a molten state; and
C. under the normal temperature of 0°C-60°C, soluble in water.

The above-mentioned medium composition is coated on the base fabric and is in a solidified waxy state without a drying process, and a finished product is obtained.

The present invention selects the following materials after a large number of experiments to meet the above-mentioned property requirements of the medium: polyglyceryl-10 stearate, dipentaerythrityl tetrahydroxystearate/tetraisostearate, potassium cetyl phosphate, hydroxyethyl acrylate/sodium acryloyldimethyl taurate copolymer, sodium stearoyl glutamate, modified Arabic gum/Xanthan gum mixture, polyethylene glycol, cholesteryl hydroxystearate, glyceryl hydroxystearate, caprylic/capric/myristic/stearic triglyceride, dipentaerythrityl hexahydroxystearate, polyglyceryl-10 oleate, hydrogenated palm oil, petrolatum, and the like.

One or more of the above substances can be selected as a medium component of the modified fabric product.

Further, in order to improve the effect of medium coating, the above-mentioned medium can be dissolved or dispersed in a medium solvent. Therefore, the present invention further provides a modified fabric product having a surface coated with a medium composition; the modified fabric product includes a base fabric and a medium composition coated on the base fabric. The medium composition includes the medium and a medium solvent capable of dissolving or dispersing the medium.

The medium solvent is selected from liquid components that can dissolve or disperse the medium at room temperature or a high temperature (0°C-500°C), and the medium solvent further has the following properties:
A. free of water or substantially free of water; and
B. in a liquid state with fluidity.

The above "A. free of water or substantially free of water" means that the water content is controlled at less than or equal to 10%, and 10% is the mass ratio.

The medium solvent includes one or a combination of an alcohol, an oil and fat, an alkane, an animal and plant extract, an emulsifier, a surfactant, a thickener, and a humectant; wherein the alcohol is selected from ethanol or a polyhydric alcohol, and the polyhydric alcohol is selected from one or more of glycerol, propylene glycol, butylene glycol, pentylene glycol, caprylyl glycol, dipropylene glycol, and panthenol; the oil and fat is selected from one or more of hydrogenated polydecene, caprylic/capric triglyceride, triethylhexanoin, vitamin E, and linoleic acid; the alkane is selected from one or more of isohexadecane, octadecane, eicosane, C14-19 alkane, C15-23 alkane; the animal and plant extract is selected from one or more of squalane, olive oil, and the like; the surfactant is selected from one or more of Tween, Span, and the like; the thickener refers exclusively to a liquid thickening component, such as a dendrobium polysaccharide solution, a polygonatum polysaccharide solution and a fructan solution; and the humectant is selected from a polyhydric alcohol or a polysaccharide.

The medium solvent is preferably one of the glycerol or the hydrogenated polydecene, or a mixture of the glycerol and the hydrogenated polydecene.

A mass ratio of the medium to the medium solvent is 1:100-100:1, preferably 1:50-50:1, 1:30-30:1, 1:10-10:1, more preferably 1:5-5 :1, 1:3-3:1.

The present invention further provides a production method of the modified fabric product having the surface coated with the medium composition, including one of the following processes:
1) melting the medium at a temperature above its melting point, and then coating directly on a surface of the base fabric;
2) melting the medium at a temperature above its melting point, dissolving in the medium solvent and mixing uniformly, and then coating on a surface of the base fabric;
3) dissolving the medium in the medium solvent and mixing uniformly, and coating on a surface of the base fabric; and
4) dispersing the medium in the medium solvent and mixing uniformly, and coating on a surface of the base fabric.

The base fabric is selected from one of a single fiber or blended spunlaced non-woven fabric, a spunlaced non-woven fabric containing seaweed fibers, a colored non-woven fabric containing carbon fibers, and a printed colored non-woven fabric. The base fabric is a pure fabric or a modified fabric that has been treated to add skin care ingredients.

A spray coating ratio of the medium composition is 0.01 g/m2-1000 g/m2, preferably 0.1 g/m2-500 g/m2, more preferably 1 g/m2-200 g/m2.

In order to meet the requirements of product efficacy, an active ingredient is further added into the modified fabric product having the surface coated with the medium composition of the present invention, such as adding the active ingredient to the base fabric, or adding the active ingredient to the medium composition, such as adding the active ingredient to the medium or the medium solvent in the medium composition. The active ingredient is selected from one or more of a chemical pharmaceutical ingredient, a traditional Chinese medicine ingredient, a bioactive ingredient, a skin beneficial ingredient, a trace element and a natural extract.

When it is necessary to add the active ingredient to the modified fabric, there are three ways to add the active ingredient. The first way is: adding the active ingredient to the base fabric, drying as needed, and then processing according to the above-mentioned production method of the modified fabric. The second way is: mixing the active ingredient with the medium, or with the medium and the medium solvent, and then processing according to the above-mentioned production method of the modified fabric. The third way is: spraying the active ingredient directly on the medium composition after coating the medium composition (generally, a medium composition without active ingredients).

However, in the pursuit of optimizing the efficacy of facial mask products, different types of active ingredients need to be added, and some ingredients work well when used in combination, but their performance will be unstable when mixed and stored, thus affecting their active efficacy. If there are strong antioxidant active substances mixed with polysaccharides and proteins, the activity will be reduced; various acidic substances mixed with proteins will also lead to the decrease of activity; proteins mixed with "carbonyl compounds" or proteins mixed with saccharides will produce non-enzymatic glycosylation reactions, which will affect the efficacy of active substances. Specifically, for example, Vitamin C (VC) and sodium hyaluronate will undergo a complex reaction to form a new substance, but the new substance is unstable; VC and glutathione will promote the discoloration of the glutathione; VC and ferulic acid will promote the discoloration of the ferulic acid. In addition, some active ingredients cannot be mixed and stored with most active substances due to their special properties. For example, highly active super antioxidant VA and poorly soluble ferulic acid or baicalin are preferably stored separately; commonly used protein skin care ingredients such as papain, collagen, and peptides are also extremely susceptible to denaturation.

In order to solve the problem of unstable efficacy after the active ingredients are mixed, the present invention further develops a multi-layer modified fabric having a surface coated with a medium composition.

A multi-layer modified fabric having a surface coated with a medium composition, the modified fabric includes a base fabric and a multi-layer medium composition coated on the base fabric; each layer of the medium composition includes a medium, or includes a medium and a medium solvent capable of dissolving or dispersing the medium; and a component of each layer of the medium composition is the same or different. The medium composition is the above-mentioned medium composition developed by the present invention.

The medium composition may or may not contain an active ingredient; active ingredients with unstable efficacy after being mixed are arranged in different layers of medium composition layers.

Each layer in the multi-layer medium composition can form a solidified waxy layer independently, which is immiscible with other layers and does not interfere with each other in activity. Therefore, each layer of the medium composition layers in the multi-layer medium composition can be used as a multi-effect functional layer or as an isolation layer. Numerous functional modified fabric products can be flexibly designed according to the efficacy requirements of the products, without being restricted by the functional stability of the active ingredients of the products, which greatly improves the market space of the products.

Based on the characteristics of the medium composition, there are three ways to add an active ingredient. The first way is: adding the active ingredient to the base fabric, drying as needed, and then processing according to the above-mentioned production process of the medium composition (in this case, the medium composition is a multifunctional layer). The second way is: mixing the active ingredient with the medium, or with the medium and the medium solvent, and then processing according to the above-mentioned production process of the medium composition (in this case, the medium composition is a multifunctional layer or an isolation layer). The third way is: spraying the active ingredient directly on the medium composition after coating the medium composition (generally, a medium composition without active ingredients) according to the above-mentioned production process (in this case, the medium composition is an isolation layer).

Based on the above invention, the present invention develops at least three types of multi-layer modified fabric products as follows.

The first type is a common moisturizing skin care mask. Each layer coating of the fabric product is a medium composition layer, but each layer coating does not contain active ingredients. The dosages of a thickener and a moisturizer in each layer, as well as the number of layers, can be set in terms of adjusting skin feeling and moisturizing properties.

The second type is a multi-effect skin care mask. Each layer coating of the fabric product is a medium composition layer, and medium composition layers are two or more layers. Active ingredients are added according to efficacy requirements. The active ingredients can be directly added to the base fabric, or mixed and coated with the medium, or the medium and the medium solvent, to form the medium composition layers; and the active ingredients with unstable efficacy after being mixed are arranged in different medium composition layers. The active ingredients can also be sprayed on the surface of the medium composition after coating the medium composition.

The third type is a universal fabric product. The medium composition layer of the present invention can be used as a "universal" additional efficacy layer or an isolation layer, which can be added to existing dry fabric products at will, and can be used as any middle layer or the outermost layer of the existing dry fabric products. The "existing common dry fabric product" can be a nearly water-free facial mask that has been dried or not dried at will, especially an existing dry fabric in existing dry fabrics requires different production processes without considering the use of the medium composition layer involved in the present invention. In this way, not only the limitation of material selection of the medium composition of the present invention is broken, but also the efficiency and application range of the existing common dry fabric products can be rapidly expanded. Accordingly, the present invention further relates to a dry fabric coated with the medium composition of the present invention, which is arranged as any one or more layers, and can be coated adjacently or at intervals.

In the production method of the multi-layer modified fabric having the surface coated with the medium composition, the process of each layer of the medium composition layers is carried out according to the production process of the mono-layer modified fabric having the surface coated with the medium composition of the present invention.

There are two ways to add the active ingredient in the multi-layer modified fabric. The first way is: adding the active ingredient to the base fabric, drying as needed, and then processing according to the production process of the medium composition in the above-mentioned modified fabric. The second way is: mixing the active ingredient with the medium, or with the medium and the medium solvent, and then processing according to the production process of the medium composition in the above-mentioned modified fabric.

The modified fabric product having the surface coated with the medium composition and the modified fabric product having the surface coated with the multi-layer medium composition of the present invention are suitable for the fields of cosmetics, daily necessities, medicines, medical apparatus, etc., such as facial mask type skin care products, wet tissue type cleaning skin care products, medical dressings, and medicine dressings.

The advantages of the present invention are as follows.

The modified fabric product having the surface coated with the medium composition and multi-layer modified fabric product having the surface coated with the medium composition of the present invention have the technical advantages as follows.
(1) After being made into finished products and before being used by consumers, the modified fabric and multi-layer modified fabric products are in a dry state with no or substantially no water, such as the water contents are controlled below 10%; or the water contents and the amounts of alcohols are controlled in a certain ratio, so that the finished products can meet the requirements of antiseptic standards during the storage period.
(2) The modified fabric and multi-layer modified fabric products have the advantages of no additives, long shelf life, stable and undamaged functional ingredients, automatic production to prevent pollution, ready-to-use and uniform dissolution.
(3) The production process has the advantages of low energy consumption and short time, which greatly reduce production costs.
(4) The surface-coated medium composition layer of the present invention can adapt to product designs with various efficacy requirements, can effectively protect the efficacy of various active ingredients, and can increase the content of efficacy ingredients according to the requirements of maximum efficacy. It not only breaks the limitation of the material selection of the medium composition of the present invention, but also can very rapidly expand the efficacy and application range of the existing common dry fabric products. Moreover, the modified fabric and multi-layer modified fabric products of the present invention have low production costs and wide market adaptability, thus having a great market value.

### DETAILED DESCRIPTION OF THE EMBODIMENTS

The present invention is further illustrated by the following embodiments, but the present invention is not limited thereto.

### Embodiment 1:

100g of potassium cetyl phosphate raw material is weighed, heated to a molten state, and 10g of *Zanthoxylum bungeanum* fruit extract is added, mixed and stirred uniformly to obtain a mixture.

The mixture is coated on the surface of a non-woven fabric according to a ratio of 1g/m², then cut into a facial mask product with a firming effect, followed with sealing and packaging.

When in use, one piece of the mask fabric with a regular face shape is placed in 25mL of purified water, fully dissolved, and then applied on the face.

### Embodiment 2:

100g of *Panax ginseng* root extracting solution raw material is weighed, sprayed uniformly on a surface of a facial mask fabric containing 20% seaweed fibers according to a ratio of 0.4g/m², and dried and dehydrated for subsequent use.

150g of sodium stearoyl glutamate is weighed and heated to a molten state to obtain a mixture. The mixture is coated on the surface of a spunlaced non-woven fabric according to a ratio of 1000 g/m², and cut into a crescent-shaped eye mask, and then sealed and packaged into an eye mask product with cleaning and moisturizing effects.

When in use, one piece of the product is placed in 12mL of purified water, fully dissolved, and then applied to the eyes.

### Embodiment 3:

100g of dipentaerythrityl tetrahydroxystearate/tetraisostearate raw material is weighed, heated to a molten state, and 10g of *Zanthoxylum bungeanum* fruit extract is added, mixed and stirred uniformly to obtain a mixture.

The mixture is coated on a surface of a non-woven fabric according to a ratio of 200g/m², then cut into a facial mask product with a firming effect, followed with sealing and packaging.

When in use, one piece of the scissor-shaped eye mask fabric is placed in 10mL of purified water, fully dissolved, and then applied to the eyes.

### Embodiment 4:

100g of *Glycyrrhiza uralensis* (licorice) root extract raw material is weighed, sprayed uniformly on a surface of a seaweed mask fabric according to a ratio of 0.4g/m², and dried and dehydrated for subsequent use.

150g of polyglyceryl-10 stearate is weighed and heated to a molten state to obtain a mixture. The mixture is coated on the surface of a spunlaced non-woven fabric according to a ratio of 500g/m², and cut into a crescent-shaped eye mask, and then sealed and packaged into an eye mask product with whitening and moisturizing effects.

When in use, one piece of the product is placed in 12mL of purified water, fully dissolved, and then applied to the eyes.

### Embodiment 5:

10g of potassium cetyl phosphate raw material is weighed, heated to a molten state, and 100g of glycerol and 1 kg of *Agave tequilana* extract raw materials are added, mixed and stirred uniformly to obtain a mixture.

The mixture is coated on the surface of a silk mask fabric according to a ratio of 30g/m², then cut into a facial mask product with a firming effect, followed with sealing and packaging.

When in use, one piece of the mask fabric printed with a panda pattern is placed in 25mL of purified water, fully dissolved, and then applied on the face.

### Embodiment 6:

100g of cholesteryl hydroxystearate raw material is weighed, heated to a molten state, and 1g of hydrogenated polydecene and 0.5kg of pearl powder raw materials are added, mixed and stirred uniformly to obtain a mixture.

The mixture is coated on the surface of a tencel fabric according to a ratio of 50g/m², then cut into a facial mask product with firming and whitening effects, followed with sealing and packaging.

When in use, one piece of the mask fabric printed with a panda pattern is placed in 25mL of purified water, fully dissolved, and then applied on the face.

### Embodiment 7:

5g of dipentaerythrityl tetrahydroxystearate/tetraisostearate and 5g of glyceryl hydroxystearate raw materials are weighed, heated to a molten state, and 500g of glycerol and 100g of sodium hyaluronate raw materials are added, mixed and stirred uniformly to obtain a mixture.

The mixture is coated on the surface of a plant fiber mask fabric according to a ratio of 20g/m², then cut into a facial mask product with a moisturizing effect, followed with sealing and packaging.

When in use, one piece of the mask fabric printed with a rose pattern is placed in 30mL of purified water, fully dissolved, and then applied on the face.

### Embodiment 8:

10g of caprylic/capric/myristic/stearic triglyceride raw material is weighed, heated to a molten state, and 1000g of butanediol and 100 million of single probiotic raw materials are added, mixed and stirred uniformly to obtain a mixture.

The mixture is coated on the surface of a spunlaced non-woven fabric according to a ratio of 15g/m², then cut into a facial mask product with a healing effect, followed with sealing and packaging.

When in use, one piece of the mask fabric is placed in 25mL of purified water, fully dissolved, and then applied to a target area.

### Embodiment 9:

300g of raw materials of dipentaerythrityl hexahydroxystearate and polyglyceryl-10 oleate are weighed according to a ratio of 2:1, heated to a molten state, and 10g of glycerol and 1kg of *Agave tequilana* extract raw materials are added, mixed and stirred uniformly to obtain a mixture.

The mixture is coated on the surface of a fruit and vegetable fiber mask fabric according to a ratio of 5g/m², then cut into a facial mask product with a firming effect, followed with sealing and packaging.

When in use, one piece of the mask fabric printed with a cartoon pattern is placed in 25mL of purified water, fully dissolved, and then applied on the face.

### Embodiment 10:

10g of raw materials of hydrogenated palm oil and polyglyceryl-10 stearate are weighed according to a ratio of 1:2, heated to a molten state, and 300g of glycerol and 1kg of *Agave tequilana* extract raw materials are added, mixed and stirred uniformly to obtain a mixture.

The mixture is coated on the surface of a non-woven fabric containing 8% seaweed fibers according to a ratio of 8g/m², then cut into a facial mask product with a firming effect, followed with sealing and packaging.

When in use, one piece of the mask fabric printed with *Agave tequilana* leaves is placed in 25mL of purified water, fully dissolved, and then applied on the face.

### Embodiment 11:

100g of petrolatum raw material is weighed, heated to a molten state, and 100g of propylene glycol and 1kg of *Agave tequilana* extract raw materials are added, mixed and stirred uniformly to obtain a mixture.

The mixture is coated on the surface of a copper ammonia fiber non-woven fabric according to a ratio of 10g/m², then cut into a facial mask product with a firming effect, followed with sealing and packaging.

When in use, one piece of the mask fabric is placed in 25mL of purified water, fully dissolved, and then applied on the face.

### Embodiment 12:

300g of polyglyceryl-10 stearate raw material is weighed, heated to a molten state, and 100g of isomeric butanediol and 1kg of *Agave tequilana* extract raw materials are added, mixed and stirred uniformly to obtain a mixture.

The mixture is positioned and coated on the surface of a pure cotton non-woven fabric according to a ratio of 25g/m² and a shape of the *Agave tequilana* leaf, then cut into a facial mask product with a firming effect, followed with sealing and packaging.

When in use, one piece of the mask fabric is placed in 25mL of purified water, fully dissolved, and then applied on the face.

### Embodiment 13:

30g of polyglyceryl-10 stearate raw material is weighed, heated to a molten state, and 150g of propylene glycol and 1kg of *Agave tequilana* extract raw materials are added, mixed and stirred uniformly to obtain a mixture.

The mixture is positioned and coated on the surface of a spunlaced non-woven fabric according to a ratio of 25g/m² and a circular shape. When cutting, the coating position is located on the nasolabial folds of the face. After cutting, a facial mask product with a firming effect is formed, then followed with sealing and packaging.

When in use, one piece of the mask fabric printed with a panda pattern is placed in 25mL of purified water, fully dissolved, and then applied on the face.

### Embodiment 14:

30g of polyglyceryl-10 stearate raw material is weighed, heated to a molten state, and 60g of isomeric butanediol and 1 kg of *Agave tequilana* extract raw materials are added, mixed and stirred uniformly to obtain a mixture.

The mixture is positioned and coated on the surface of a spunlaced non-woven fabric according to a ratio of 10g/m² and a linear shape. When cutting, the coating position is located on the forehead wrinkles. After cutting, a facial mask product with a firming effect is formed, then followed with sealing and packaging.

When in use, one piece of the mask fabric printed with a panda pattern is placed in 25mL of purified water, fully dissolved, and then applied on the face.

### Embodiment 15:

300g of polyglyceryl-10 stearate raw material is weighed, heated to a molten state, and 150g of isomeric butanediol and 1kg of *Agave tequilana* extract raw materials are added, mixed and stirred uniformly to obtain a mixture.

The mixture is coated on the surface of a spunlaced non-woven fabric according to a ratio of 12.5g/m², and coated according to a triangle shape with a two-sided positioning. When cutting, the coating position is located on the corner of the eye to form a facial mask product with tight efficacy of eye striae; then followed with sealing and packaging.

When in use, one piece of the mask fabric printed with a panda pattern is placed in 25mL of purified water, fully dissolved, and then applied on the face.

### Embodiment 16:

100g of p-chloro-xylenol and 100g of hydroxyethyl acrylate/sodium acryloyldimethyl taurate copolymer raw materials are weighed, dissolved in 300g of alcohol, and sprayed uniformly on the surface of a facial mask fabric containing 2% seaweed fibers at a ratio of 50g/m², and then air-dried naturally in a ten thousand grade clean workshop until the moisture content is less than 5%.

The processed facial mask fabric is cut into an oval shape of 8x16cm, sealed and packaged into sterile paper towels.

When in use, one piece of the sterile paper towels is fully dissolved with 8mL of purified water, and then a target part is wiped with the sterile paper towel for efficient disinfection and sterilization.

### Embodiment 17:

50g of ginsenoside extract, 5g of dipentaerythrityl tetrahydroxystearate/tetraisostearate, 5g of hydroxyethyl acrylate/sodium acryloyldimethyl taurate copolymer raw materials are weighed and dissolved in 100g of alcohol, sprayed uniformly on the surface of a seaweed mask fabric at a ratio of 0.1g/m², and air-dried naturally.

The processed seaweed mask fabric is cut into a rectangle shape of 5x20cm, sealed and packaged into a product.

When in use, one piece of the product is fully dissolved with 8mL of purified water, and then applied to a target part for external use.

### Embodiment 18:

500g of ginsenoside extract, 1g of dipentaerythrityl tetrahydroxystearate/tetraisostearate, 1g of hydroxyethyl acrylate/sodium acryloyldimethyl taurate copolymer raw materials are weighed and dissolved in 100g of alcohol, sprayed uniformly on the surface of a seaweed mask fabric at a ratio of 150g/m², and air-dried naturally.

The processed seaweed mask fabric is cut into a rectangle shape of 15x30cm, sealed and packaged into plaster-type external medicine.

When in use, one piece of the plaster is fully dissolved with 8mL of purified water, and then applied to a target part.

### Embodiment 19:

10 g of Xanthan gum and 20g of ascorbic acid powder are weighed, dispersed in 10g of alcohol, sprayed uniformly on the surface of a spunlaced non-woven fabric at a ratio of 0.01g/m², and air-dried naturally.

The processed spunlacedd non-woven fabric is cut into a face shape to form a facial mask product with a skin brightening effect, then followed with sealing and packaging.

When in use, one piece of the facial mask fabric is fully dissolved with 25mL of purified water, and then applied to the face.

### Embodiment 20:

10g of Arabic gum and 10µg of epidermal growth factor (EGF) freeze-dried powder are weighed, dispersed in 20g of alcohol, and sprayed uniformly on the surface of a spunlaced non-woven fabric at a ratio of 0.1g/m², and then air-dried naturally.

The processed spunlaced non-woven fabric is cut into medicinal dressings with a skin wound repairing effect, then followed with sealing and packaging.

When in use, one piece of the medicinal dressings is fully dissolved with 25mL of purified water, and then applied to the face.

### Embodiment 21:

10g of caprylic/capric/myristic/stearic triglyceride raw material is weighed, heated to a molten state to obtain a mixture.

The mixture is coated on the surface of a silk mask fabric at a ratio of 0.01g/m², and then 1g of a papain solution is sprayed on the surface of the silk mask fabric.

The processed silk mask fabric is cut into a facial mask product with a firming effect, followed with sealing and packaging.

When in use, one piece of the mask fabric printed with a panda pattern is placed in 25mL of purified water, fully dissolved, and then applied on the face.

### Embodiment 22:

100g of potassium cetyl phosphate raw material is weighed and then heated to a molten state as a first-layer medium composition.

200g of raw materials of dipentaerythrityl hexahydroxystearate and butanediol are weighed according to a ratio of 1:3 and then heated to a molten state as a second-layer medium composition.

100g of hydroxyethyl acrylate/sodium acryloyldimethyl taurate copolymer raw material is weighed and dissolved in 300g of alcohol as a third-layer medium composition.

The first-layer medium composition, the first-layer medium composition, and the third-layer medium composition are sequentially coated on a spunlaced non-woven fabric; a coating amount of the first-layer medium composition is 1g/m², a coating amount of the second-layer medium composition is 200g/m²; and the third-layer medium composition is sprayed uniformly on the surface of the mask fabric containing 2% seaweed fibers at a ratio of 50g/m²; and then the facial mask fabric is air-dried naturally in a ten thousand grade clean workshop until the moisture content is less than 5%.

The coated mask fabric is cut into a facial mask with a strong moisturizing function, followed with sealing and packaging.

When in use, one piece of the mask fabric is placed in 25mL of purified water, fully dissolved, and then applied on the face.

### Embodiment 23:

100g of potassium cetyl phosphate raw material is weighed, heated to a molten state, and 10 g of VC is added, and then mixed and stirred uniformly, as a first-layer medium composition.

100g of polyglyceryl-10 stearate is weighed, heated to a molten state, and 5g of a ferulic acid-disodium hydrogen phosphate mixed solution is added, as a second-layer medium composition.

The first-layer medium composition and the first-layer medium composition are sequentially coated on a spunlaced non-woven fabric. A coating amount of the first-layer medium composition is 50g/m², and a coating amount of the second-layer medium composition is 100g/m². The coated mask fabric is cut into a facial mask with a strong whitening function, followed with sealing and packaging.

When in use, one piece of the mask fabric with a regular face shape is placed in 25mL of purified water, fully dissolved, and then applied on the face.

### Embodiment 24:

10g of *Sophora angustifolia* root extract raw material is weighed, sprayed uniformly on the surface of a mask fabric containing 20% seaweed fibers according to a ratio of 0.4g/m², and dried and dehydrated for subsequent use.

150g of sodium stearoyl glutamate raw material is weighed and heated to a molten state as a first-layer medium composition.

10g of baicalin is weighed, dispersed in 20g of alcohol, sprayed uniformly on the surface of the spunlaced non-woven fabric at a ratio of 0.1g/m², and air-dried naturally as a second-layer medium composition.

The first-layer medium composition and the second-layer medium composition are sequentially coated on the spunlaced non-woven fabric; the first-layer medium composition is coated on the surface of the spunlaced nonwoven fabric at a ratio of 1000 g/m², and the first-layer medium composition is coated on the surface of the spunlaced nonwoven fabric at a ratio of 0.1g/m². After the second-layer the medium composition is coated, the coated fabric is air-dried naturally, and then cut into a mask product with strong moisturizing and acne removing functions, followed with sealing and packaging.

When in use, one piece of the mask fabric is placed in 25mL of purified water, fully dissolved, and then applied on the face.

### Embodiment 25:

10g of hydroxyethyl acrylate/sodium acryloyldimethyl taurate copolymer is weighed, heated to a molten state, and hydrolyzed sodium hyaluronate is added and mixed uniformly, as a first-layer medium composition.

150g of sodium stearoyl glutamate raw material is weighed, heated to a molten state, glutathione is added and mixed uniformly, as a second-layer medium composition.

10g of papain is weighed and dispersed in 20g of alcohol, as a third-layer medium composition.

10g of a modified Arabic gum/Xanthan gum mixture is weighed and heated to a molten state, as a fourth-layer medium composition.

The first-layer medium composition, the first-layer medium composition, the third-layer medium composition, and the fourth-layer medium composition are sequentially coated on a colored non-woven fabric containing carbon fibers, and a coating amount is adjustable from 0.01g/m² to 1000g/m². Then the coated fabric is cut into a powerful and multifunctional fabric product, followed with sealing and packaging.

When in use, one piece of the mask fabric is placed in 25mL of purified water, fully dissolved, and then applied on the face.

### Embodiment 26:

100g of polyglyceryl-10 oleate is weighed and heated to a molten state as a first-layer medium composition.

100g of a modified Arabic gum/Xanthan gum mixture is weighed, heated to a molten state, and 10g of hydrogenated polydecene is added and mixed uniformly, as a second-layer medium composition.

The first-layer medium composition is coated on a colored non-woven fabric containing carbon fibers at a ratio of 10g/m², and 20g of a hexapeptide-2 solution is sprayed, and then the second-layer medium composition is coated on the fabric at a ratio of 50g/m².

The coated fabric is cut into a facial mask product with a freckle removing effect, followed with sealing and packaging.

When in use, one piece of the mask fabric is placed in 25 mL of purified water, fully dissolved, and then applied on the face.

### Embodiment 27:

100g of *Aesculus hippocastanum* (Horse chestnut) seed extract raw material is weighed, sprayed uniformly on the surface of a mask fabric containing 20% seaweed fibers according to a ratio of 0.4g/m², and dried and dehydrated for subsequent use.

5g of dipentaerythrityl tetrahydroxystearate/tetraisostearate, 5g of glyceryl hydroxystearate raw materials are weighed, heated to a molten state, and 500g of glycerol and 100g of hydrolyzed rice protein raw materials are added, mixed and stirred uniformly to obtain a mixture.

The mixture is coated on the surface of the spunlaced non-woven fabric according to a ratio of 20g/m², and cut into a crescent-shaped eye mask, and then sealed and packaged into an eye mask product with cleaning and moisturizing effects.

When in use, one piece of the product is placed in 12mL of purified water, fully dissolved, and then applied to the eyes.

### Embodiment 28:

Preparation of a common moisturizing dry fabric: hyaluronic acid is stirred and dissolved with water to prepare a 1% high-molecular polymer aqueous solution. The aqueous solution is sprayed on a prepared cotton fabric, and then putted in an oven for drying to remove moisture to obtain a pre-coated high-molecular polymer fabric, and a ratio of the hyaluronic acid to the fabric is 10g/m².

Preparation of a medium composition: 100g of cholesteryl hydroxystearate raw material is weighed, heated to a molten state, 100g of carnosine is added, and mixed and stirred uniformly.

The medium composition is coated on the common moisturizing dry fabric at a ratio of 1000g/m², and then cut into a facial mask product with strong moisturizing and freckle removal functions, followed with sealing and packaging.

When in use, one piece of the mask fabric is placed in 25mL of purified water, fully dissolved, and then applied on the face.

### Embodiment 29:

A new coating is added to the common moisturizing dry fabric of Embodiment 26: tremella polysaccharide is stirred and dissolved with water to prepare a 0.8% high-molecular polymer aqueous solution. The aqueous solution is sprayed a prepared colored non-woven fabric with printed patterns, and then putted in an oven for drying to remove moisture to obtain a pre-coated high-molecular polymer fabric, and a ratio of the tremella polysaccharide to the fabric is 5g/m².

Preparation of a medium composition: 10g of caprylic/capric/myristic/stearic triglyceride raw material is weighed, heated to a molten state, 200g of glycerol, 200g of hydrogenated polydecene and 1g of bifida ferment lysate are added, mixed and stirred uniformly; a coating amount of the medium composition is 200g/m².

The coating layer containing tremella polysaccharide and the medium composition layer can be used as a second coating layer or a third coating layer. After coating, the fabric is cut into a facial mask product with a strong moisturizing function, followed with sealing and packaging.

When in use, one piece of the mask fabric is placed in 25mL of purified water, fully dissolved, and then applied on the face.

### Embodiment 30:

200g of p-chloro-xylenol and 300g of hydroxyethyl acrylate/sodium acryloyldimethyl taurate copolymer are weighed, dissolved in 10g of alcohol, and sprayed uniformly on the surface of a facial mask fabric containing 2% seaweed fibers at a ratio of 50g/m², and then the facial mask fabric is air-dried naturally in a ten thousand grade clean workshop until the moisture content is less than 5%.

Then, a second-layer medium composition is arranged to increase a disinfection effect: 100g of petrolatum raw material is weighed, heated to a molten state, 10g of VC is added, then mixed and stirred uniformly; a coating amount of the second-layer medium composition is 100g/m².

The processed facial mask fabric is cut into an oval shape of 8x16cm, sealed and packaged into sterile paper towels.

When in use, one piece of the sterile paper towels is taken and fully dissolved with 8mL of purified water, and then a target part is wiped with the sterile paper towel for efficient disinfection and sterilization.

### Embodiment 31:

50g of ginsenoside extract, 5g of dipentaerythrityl tetrahydroxystearate/tetraisostearate, 5g of hydroxyethyl acrylate/sodium acryloyldimethyl taurate copolymer raw materials are weighed and dissolved in 100g of alcohol, and sprayed uniformly on the surface of a seaweed mask fabric at a ratio of 0.1g/m², and air-dried naturally.

Then, a second-layer medium composition is arranged to increase a moisturizing effect: 300g of polyglyceryl-10 stearate raw material is weighed, heated to a molten state, and 60g of hydrogenated polydecene is added, mixed and stirred uniformly; a coating amount of the second-layer medium composition is 100g/m².

Then, a third-layer medium composition is arranged to increase a disinfection effect: 100g of p-chloro-xylenol and 100g of hydroxyethyl acrylate/sodium acryloyldimethyl taurate copolymer raw materials are weighed, dissolved in 300g of alcohol, and sprayed uniformly on the surface of a facial mask fabric containing 2% seaweed fibers at a ratio of 50g/m², and then the facial mask fabric is air-dried naturally in a ten thousand grade clean workshop until the moisture content is less than 5%.

The processed fabric is cut into a rectangle shape of 5×20cm, sealed and packaged into a product.

When in use, one piece of the product is fully dissolved with 8mL of purified water, and then applied to a target area for external use.

The components, form and preparation method of the present invention are not limited to the forms listed in the embodiments. The embodiments are merely preferred embodiments of the present invention, which are not used to limit the protective scope. Any simple or equivalent variations and modifications within the scope of the claims of the present invention shall fall within the protective scope of the present invention.

## Claims

1. A modified fabric having a surface coated with a medium composition, comprising a base fabric and a medium composition coated on the base fabric; wherein the medium composition comprises a medium, or comprises the medium and a medium solvent capable of dissolving or dispersing the medium; and the medium simultaneously has the following properties:
A. under a normal temperature of 0°C-60°C, in a solidification state;
B. under a high temperature of 30°C-500°C, in a molten state; and
C. under the normal temperature of 0°C-60°C, soluble in water.

2. The modified fabric according to claim 1, wherein, the medium is at least one selected from the group consisting of polyglyceryl-10 stearate, dipentaerythrityl tetrahydroxystearate/tetraisostearate, potassium cetyl phosphate, hydroxyethyl acrylate/sodium acryloyldimethyl taurate copolymer, sodium stearoyl glutamate, modified Arabic gum/Xanthan gum mixture, polyethylene glycol, cholesteryl hydroxystearate, glyceryl hydroxystearate, caprylic/capric/myristic/stearic triglyceride, dipentaerythrityl hexahydroxystearate, polyglyceryl-10 oleate, hydrogenated palm oil, and petrolatum.

3. The modified fabric according to claim 1, wherein, the medium solvent is selected from liquid components capable of dissolving or dispersing the medium at a normal temperature or a high temperature ranging from 0°C to 500°C, and the medium solvent further has the following properties:
A. free of water or substantially free of water; and
B. in a liquid state with fluidity.

4. The modified fabric according to claim 3, wherein, the medium solvent is "free of water or substantially free of water" means that a mass fraction of the water content of the medium solvent is less than or equal to 10%.

5. The modified fabric according to claim 3, wherein, the medium solvent is at least one selected from the group consisting of an alcohol, an oil and fat, an alkane, an animal and plant extract, an emulsifier, a surfactant, a thickener, and a humectant.

6. The modified fabric according to claim 5, wherein, in the medium solvent, the alcohol is at least one selected from the group consisting of ethanol and a polyhydric alcohol, and the polyhydric alcohol is at least one selected from the group consisting of glycerol, propylene glycol, butylene glycol, pentylene glycol, caprylyl glycol, dipropylene glycol, and panthenol; the oil and fat is at least one selected from the group consisting of hydrogenated polydecene, caprylic/capric triglyceride, triethylhexanoin, vitamin E, and linoleic acid; the alkane is at least one selected from the group consisting of isohexadecane, octadecane, eicosane, C14-19 alkane, and C15-23 alkane; the animal and plant extract is at least one selected from the group consisting of squalene and olive oil; the surfactant is at least one selected from the group consisting of Tween and Span; the thickener refers to a liquid thickening component; and the humectant is selected from a polyhydric alcohol or a polysaccharide.

7. The modified fabric according to claim 6, wherein the medium solvent is the glycerol or the hydrogenated polydecene, or a mixture of the glycerol and the hydrogenated polydecene.

8. The modified fabric according to claim 1, wherein, a mass ratio of the medium to the medium solvent is 1:100-100:1, preferably 1:50-50:1, 1:30-30:1, 1:10-10:1, and more preferably 1:5-5:1, 1:3-3:1.

9. The modified fabric according to claim 1, wherein, an active ingredient is added to the base fabric, or the active ingredient is added to the medium composition.

10. The modified fabric according to claim 9, wherein, the active ingredient is at least one selected from the group consisting of a chemical pharmaceutical ingredient, a traditional Chinese medicine ingredient, a bioactive ingredient, a skin beneficial ingredient, a trace element and a natural extract.

11. The modified fabric according to claim 1, wherein, a production method of the modified fabric comprises one of the following processes:
1) melting the medium at a temperature above a melting point of the medium, and then coating directly on a surface of the base fabric;
2) melting the medium at a temperature above a melting point of the medium, dissolving in the medium solvent and mixing uniformly, and then coating on a surface of the base fabric;
3) dissolving the medium in the medium solvent and mixing uniformly, and coating on a surface of the base fabric; and
4) dispersing the medium in the medium solvent and mixing uniformly, and coating on a surface of the base fabric.

12. The modified fabric according to claim 11, wherein, when it is necessary to add an active ingredient to the modified fabric, there are three ways to add the active ingredient; a first way is: adding the active ingredient to the base fabric, and then processing according to the production method of the modified fabric; a second way is: mixing the active ingredient with the medium, or with the medium and the medium solvent, and then processing according to the production method of the modified fabric; and a third way is: spraying the active ingredient directly on the medium composition after coating the medium composition.

13. A production method of a modified fabric having a surface coated with a medium composition, comprising one of the following processes:
1) melting the medium at a temperature above a melting point of the medium, and then coating directly on a surface of the base fabric;
2) melting the medium at a temperature above a melting point of the medium, dissolving in the medium solvent and mixing uniformly, and then coating on a surface of the base fabric;
3) dissolving the medium in the medium solvent and mixing uniformly, and coating on a surface of the base fabric; and
4) dispersing the medium in the medium solvent and mixing uniformly, and coating on a surface of the base fabric.

14. The production method according to claim 13, wherein, when it is necessary to add an active ingredient to the modified fabric, there are three ways to add the active ingredient; a first way is: adding the active ingredient to the base fabric, and then processing according to the production method of the modified fabric; a second way is: mixing the active ingredient with the medium, or with the medium and the medium solvent, and then processing according to the production method of the modified fabric; and a third way is: spraying the active ingredient directly on the medium composition after coating the medium composition.

15. The modified fabric according to claim 1, wherein, the base fabric is one selected from the group consisting of a single fiber or blended spunlaced non-woven fabric, a spunlaced non-woven fabric containing seaweed fibers, a colored non-woven fabric containing carbon fibers, a printed colored non-woven fabric, and a modified fabric.

16. The modified fabric according to claim 1, wherein, a spray coating ratio of the medium composition to the base fabric is 0.01g/m² to 1000g/m².

17. An application of the modified fabric having the surface coated with the medium composition according to claim 1 in cosmetics, daily necessities, medicines, and medical devices.

18. A medium composition layer, comprising a medium, or comprises a medium and a medium solvent capable of dissolving or dispersing the medium; wherein the medium simultaneously has the following properties:
A. under a normal temperature of 0°C-60°C, in a solidification state;
B. under a high temperature of 30°C-500°C, in a molten state; and
C. under the normal temperature of 0°C-60°C, soluble in water.

19. The modified fabric according to claim 18, wherein, the medium solvent is selected from liquid components capable of dissolving or dispersing the medium at a normal temperature or a high temperature ranging from 0°C to 500°C, and the medium solvent further has the following properties:
A. free of water or substantially free of water; and
B. in a liquid state with fluidity.

20. A multi-layer modified fabric having a surface coated with a medium composition, comprising a base fabric and a multi-layer medium composition coated on the base fabric; each layer of the medium composition comprises a medium, or comprises a medium and a medium solvent capable of dissolving or dispersing the medium; a component of each layer of medium composition is the same or different; and the medium simultaneously has the following properties:
A. under a normal temperature of 0°C-60°C, in a solidification state;
B. under a high temperature of 30°C-500°C, in a molten state; and
C. under the normal temperature of 0°C-60°C, soluble in water.

21. The modified fabric according to claim 20, wherein, the medium is at least one selected from the group consisting of polyglyceryl-10 stearate, dipentaerythrityl tetrahydroxystearate/tetraisostearate, potassium cetyl phosphate, hydroxyethyl acrylate/sodium acryloyldimethyl taurate copolymer, sodium stearoyl glutamate, modified Arabic gum/Xanthan gum mixture, polyethylene glycol, cholesteryl hydroxystearate, glyceryl hydroxystearate, caprylic/capric/myristic/stearic triglyceride, dipentaerythrityl hexahydroxystearate, polyglyceryl-10 oleate, hydrogenated palm oil, and petrolatum.

22. The modified fabric according to claim 20, wherein, the medium solvent is selected from liquid components capable of dissolving or dispersing the medium at a normal temperature or a high temperature ranging from 0°C to 500°C, and the medium solvent further has the following properties:
A. free of water or substantially free of water; and
B. in a liquid state with fluidity.

23. The modified fabric according to claim 22, wherein, the medium solvent is "free of water or substantially free of water" means that a mass fraction of the water content of the medium solvent is less than or equal to 10%.

24. The modified fabric according to claim 22, wherein, the medium solvent is at least one selected from the group consisting of an alcohol, an oil and fat, an alkane, an animal and plant extract, an emulsifier, a surfactant, a thickener, and a humectant.

25. The modified fabric according to claim 24, wherein, in the medium solvent, the alcohol is at least one selected from the group consisting of ethanol and a polyhydric alcohol, and the polyhydric alcohol is at least one selected from the group consisting of glycerol, propylene glycol, butylene glycol, pentylene glycol, caprylyl glycol, dipropylene glycol, and panthenol; the oil and fat is at least one selected from the group consisting of hydrogenated polydecene, caprylic/capric triglyceride, triethylhexanoin, vitamin E, and linoleic acid; the alkane is at least one selected from the group consisting of isohexadecane, octadecane, eicosane, C14-19 alkane, and C15-23 alkane; the animal and plant extract is at least one selected from the group consisting of squalene and olive oil; the surfactant is at least one selected from the group consisting of Tween and Span; the thickener refers to a liquid thickening component; and the humectant is selected from a polyhydric alcohol or a polysaccharide.

26. The modified fabric according to claim 25, wherein, the medium solvent is the glycerol or the hydrogenated polydecene, or a mixture of the glycerol and the hydrogenated polydecene.

27. The modified fabric according to claim 20, wherein, a mass ratio of the medium to the medium solvent is 1:100-100:1, preferably 1:50-50:1, 1:30-30:1, 1:10-10:1, and more preferably 1:5-5:1, 1:3-3:1.

28. The modified fabric according to claim 20, wherein, an active ingredient is added to the base fabric, or the active ingredient is added to the medium composition.

29. The modified fabric according to claim 28, wherein, active ingredients with unstable efficacy after being mixed are arranged in different layers of medium composition.

30. The modified fabric according to claim 28, wherein, the active ingredient is at least one selected from the group consisting of a chemical pharmaceutical ingredient, a traditional Chinese medicine ingredient, a bioactive ingredient, a skin beneficial ingredient, a trace element and a natural extract.

31. The modified fabric according to claim 20, wherein, the medium composition is applied to one of the following processes:
1) melting the medium at a temperature above a melting point of the medium, and then coating directly on a surface of the base fabric;
2) melting the medium at a temperature above a melting point of the medium, dissolving in the medium solvent and mixing uniformly, and then coating on a surface of the base fabric;
3) dissolving the medium in the medium solvent and mixing uniformly, and coating on a surface of the base fabric; and
4) dispersing the medium in the medium solvent and mixing uniformly, and coating on a surface of the base fabric.

32. The modified fabric according to claim 31, wherein, when it is necessary to add an active ingredient to the modified fabric, there are three ways to add the active ingredient; a first way is: adding the active ingredient to the base fabric, and then processing according to the production method of the modified fabric; a second way is: mixing the active ingredient with the medium, or with the medium and the medium solvent, and then processing according to the process of the medium composition; and a third way is: spraying the active ingredient directly on the medium composition after coating the medium composition.

33. A production method of a multi-layer modified fabric having a surface coated with a medium composition, wherein, the medium composition is applied to one of the following processes:
1) melting the medium at a temperature above a melting point of the medium, and then coating directly on a surface of the base fabric;
2) melting the medium at a temperature above a melting point of the medium, dissolving in the medium solvent and mixing uniformly, and then coating on a surface of the base fabric;
3) dissolving the medium in the medium solvent and mixing uniformly, and coating on a surface of the base fabric; and
4) dispersing the medium in the medium solvent and mixing uniformly, and coating on a surface of the base fabric.

34. The production method according to claim 33, wherein, when the active ingredient is added to the multi-layer modified fabric, there are three ways to add the active ingredient; a first way is: adding the active ingredient to the base fabric, and then processing according to the production method of the modified fabric; a second way is: mixing the active ingredient with the medium, or with the medium and the medium solvent, and then processing according to the process of the medium composition; and a third way is: spraying the active ingredient directly on the medium composition after coating the medium composition.

35. The modified fabric according to claim 20, wherein, the base fabric is one selected from the group consisting of a single fiber or blended spunlaced non-woven fabric, a spunlaced non-woven fabric containing seaweed fibers, a colored non-woven fabric containing carbon fibers, a printed colored non-woven fabric, and a modified fabric.

36. The modified fabric according to claim 20, wherein, a spray coating ratio of the medium composition to the base fabric is 0.01g/m²-1000g/m².

37. An application of the multi-layer modified fabric having the surface coated with the medium composition according to claim 20 in cosmetics, daily necessities, medicines, and medical devices.

38. A dry fabric, wherein the dry fabric is coated with the medium composition according to claim 1 as any one or more layers.

39. The dry fabric according to claim 38, wherein, the medium composition is coated adjacently or at intervals.
